# EUROPEAN PATENT APPLICATION

(11) **EP 3 211 422 A1**
(43) Date of publication of application: **30.08.2017**
(21) Application number: 16156886.0
(22) Date of filing: 23.02.2016
(51) Int. Cl.: G01N 33/68, A61K 49/00, G01N 33/542

(54) **METHOD FOR MEASUREMENT AND CONTROL OF INTRAOCULAR VEGF CONCENTRATION**

(71) Applicant: Justus-Liebig-Universität Gießen, 35390 Gießen (DE)
(72) Inventor: Stieger, Knut, 35415 Pohlheim (DE); Wimmer, Tobias, 35392 Giessen (DE); Lorenz, Birgit, 35321 Laubach (DE)
(74) Representative: Stumpf, Peter

(57) **Abstract**

The invention describes a new method for in vivo measurement and control of intraocular VEGF concentration using bioluminescence resonance energy transfer (BRET) of a VEGF-binding biosensor. Furthermore, the method is suitable for highly sensitive in vitro determination of VEGF concentration from a small sample volume.

SEQUENCE LISTING

<110>  Justus-Liebig-University Giessen

<120>  Method for measurement and control of intraocular VEGF
       concentration

<130>  TM 907

<160>  2

<170>  PatentIn version 3.5

<210>  1
<211>  999
<212>  PRT
<213>  Renilla reniformis

<220>
<221>  PEPTIDE
<222>  (1)..(311)
<223>  RLuc8 - Renilla luciferase mutant 8

<220>
<221>  PEPTIDE
<222>  (312)..(762)
<223>  Ra02 - single chain variable fragment (scFv) of ranibizumab

<220>
<221>  PEPTIDE
<222>  (763)..(999)
<223>  GFP2 - Green fluorescent protein variant

<400>  1

Met Ala Ser Lys Val Tyr Asp Pro Glu Gln Arg Lys Arg Met Ile Thr
1               5                   10                  15

Gly Pro Gln Trp Trp Ala Arg Cys Lys Gln Met Asn Val Leu Asp Ser
            20                  25                  30

Phe Ile Asn Tyr Tyr Asp Ser Glu Lys His Ala Glu Asn Ala Val Ile
        35                  40                  45

Phe Leu His Gly Asn Ala Thr Ser Ser Tyr Leu Trp Arg His Val Val
    50                  55                  60

Pro His Ile Glu Pro Val Ala Arg Cys Ile Ile Pro Asp Leu Ile Gly
65                  70                  75                  80

Met Gly Lys Ser Gly Lys Ser Gly Asn Gly Ser Tyr Arg Leu Leu Asp
                85                  90                  95

His Tyr Lys Tyr Leu Thr Ala Trp Phe Glu Leu Leu Asn Leu Pro Lys
            100                 105                 110

Lys Ile Ile Phe Val Gly His Asp Trp Gly Ala Ala Leu Ala Phe His
        115                 120                 125

Tyr Ala Tyr Glu His Gln Asp Arg Ile Lys Ala Ile Val His Met Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu
                165                 170                 175

Glu Asn Asn Phe Phe Val Glu Thr Val Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Leu Phe Ile Glu
                245                 250                 255

Ser Asp Pro Gly Phe Phe Ser Asn Ala Ile Val Glu Gly Ala Lys Lys
            260                 265                 270

Phe Pro Asn Thr Glu Phe Val Lys Val Lys Gly Leu His Phe Leu Gln
        275                 280                 285

Glu Asp Ala Pro Asp Glu Met Gly Lys Tyr Ile Lys Ser Phe Val Glu
    290                 295                 300

Arg Val Leu Lys Asn Glu Gln Asp Ile Gln Leu Thr Gln Ser Pro Ser
305                 310                 315                 320

Ser Leu Ser Ala Ser Val Gly Asp Arg Val Thr Ile Thr Cys Ser Ala
                325                 330                 335

Ser Gln Asp Ile Ser Asn Tyr Leu Asn Trp Tyr Gln Gln Lys Pro Gly
            340                 345                 350

Lys Ala Pro Lys Val Leu Ile Tyr Phe Thr Ser Ser Leu His Ser Gly
        355                 360                 365

Val Pro Ser Arg Phe Ser Gly Ser Gly Ser Gly Thr Asp Phe Thr Leu
    370                 375                 380

Thr Ile Ser Ser Leu Gln Pro Glu Asp Phe Ala Thr Tyr Tyr Cys Gln
385                 390                 395                 400

Gln Tyr Ser Thr Val Pro Trp Thr Phe Gly Gln Gly Thr Lys Val Glu
                405                 410                 415

Ile Lys Arg Thr Val Ala Ala Pro Ser Val Phe Ile Phe Pro Pro Ser
            420                 425                 430

Asp Glu Gln Leu Lys Ser Gly Thr Ala Ser Val Val Cys Leu Leu Asn
        435                 440                 445

Asn Phe Tyr Pro Arg Glu Ala Lys Val Gln Trp Lys Val Asp Asn Ala
    450                 455                 460

Leu Gln Ser Gly Asn Ser Gln Glu Ser Val Thr Glu Gln Asp Ser Lys
465                 470                 475                 480

Asp Ser Thr Tyr Ser Leu Ser Ser Thr Leu Thr Leu Ser Lys Ala Asp
                485                 490                 495

Tyr Glu Lys His Lys Val Tyr Ala Cys Glu Val Thr His Gln Gly Leu
            500                 505                 510

Ser Ser Pro Val Thr Lys Ser Phe Asn Arg Gly Glu Cys Gly Gly Gly
        515                 520                 525

Gly Gly Gly Glu Val Gln Leu Val Glu Ser Gly Gly Gly Leu Val Gln
    530                 535                 540

Pro Gly Gly Ser Leu Arg Leu Ser Cys Ala Ala Ser Gly Tyr Asp Phe
545                 550                 555                 560

Thr His Tyr Gly Met Asn Trp Val Arg Gln Ala Pro Gly Lys Gly Leu
                565                 570                 575

Glu Trp Val Gly Trp Ile Asn Thr Tyr Thr Gly Glu Pro Thr Tyr Ala
            580                 585                 590

Ala Asp Phe Lys Arg Arg Phe Thr Phe Ser Leu Asp Thr Ser Lys Ser
        595                 600                 605

Thr Ala Tyr Leu Gln Met Asn Ser Leu Arg Ala Glu Asp Thr Ala Val

610                 615                 620

Tyr Tyr Cys Ala Lys Tyr Pro Tyr Tyr Tyr Gly Thr Ser His Trp Tyr
625                 630                 635                 640

Phe Asp Val Trp Gly Gln Gly Thr Leu Val Thr Val Ser Ser Ala Ser
                645                 650                 655

Thr Lys Gly Pro Ser Val Phe Pro Leu Ala Pro Ser Ser Lys Ser Thr
            660                 665                 670

Ser Gly Gly Thr Ala Ala Leu Gly Cys Leu Val Lys Asp Tyr Phe Pro
        675                 680                 685

Glu Pro Val Thr Val Ser Trp Asn Ser Gly Ala Leu Thr Ser Gly Val
    690                 695                 700

His Thr Phe Pro Ala Val Leu Gln Ser Ser Gly Leu Tyr Ser Leu Ser
705                 710                 715                 720

Ser Val Val Thr Val Pro Ser Ser Ser Leu Gly Thr Gln Thr Tyr Ile
                725                 730                 735

Cys Asn Val Asn His Lys Pro Ser Asn Thr Lys Val Asp Lys Lys Val
            740                 745                 750

Glu Pro Lys Ser Cys Asp Lys Thr His Leu Ser Gly Gly Glu Glu Leu
        755                 760                 765

Phe Ala Gly Ile Val Pro Val Leu Ile Glu Leu Asp Gly Asp Val His
    770                 775                 780

Gly His Lys Phe Ser Val Arg Gly Glu Gly Glu Gly Asp Ala Asp Tyr
785                 790                 795                 800

Gly Lys Leu Glu Ile Lys Phe Ile Cys Thr Thr Gly Lys Leu Pro Val
                805                 810                 815

Pro Trp Pro Thr Leu Val Thr Thr Leu Cys Tyr Gly Ile Gln Cys Phe
            820                 825                 830

Ala Arg Tyr Pro Glu His Met Lys Met Asn Asp Phe Phe Lys Ser Ala
        835                 840                 845

Met Pro Glu Gly Tyr Ile Gln Glu Arg Thr Ile Gln Phe Gln Asp Asp
    850                 855                 860

Gly Lys Tyr Lys Thr Arg Gly Glu Val Lys Phe Glu Gly Asp Thr Leu
865                 870                 875                 880

Val Asn Arg Ile Glu Leu Lys Gly Lys Asp Phe Lys Glu Asp Gly Asn
                885                 890                 895

Ile Leu Gly His Lys Leu Glu Tyr Ser Phe Asn Ser His Asn Val Tyr
            900                 905                 910

Ile Arg Pro Asp Lys Ala Asn Asn Gly Leu Glu Ala Asn Phe Lys Thr
        915                 920                 925

Arg His Asn Ile Glu Gly Gly Gly Val Gln Leu Ala Asp His Tyr Gln
    930                 935                 940

Thr Asn Val Pro Leu Gly Asp Gly Pro Val Leu Ile Pro Ile Asn His
945                 950                 955                 960

Tyr Leu Ser Thr Gln Thr Lys Ile Ser Lys Asp Arg Asn Glu Ala Arg
                965                 970                 975

Asp His Met Val Leu Leu Glu Ser Phe Ser Ala Cys Cys His Thr His
            980                 985                 990

Gly Met Asp Glu Leu Tyr Arg
        995

<210>  2
<211>  8736
<212>  DNA
<213>  Vector

<400>  2
cagctgcgcg ctcgctcgct cactgaggcc gcccgggcaa agcccgggcg tcgggcgacc      60

tttggtcgcc cggcctcagt gagcgagcga gcgcgcagag agggagtggc caactccatc     120

actaggggtt ccttgtagtt aatgattaac ccgccatgct acttatctac gtagccatgc     180

tctagctgaa gctgatcgat ccagcttggt cgagctgata cttcccgtcc gccaggggac     240

atgccggcga tgctgaaggt cgcgcgcatt cccgatgaag aggccggtta ccgcctgttg     300

acctggtggg acgggcaggg cgccgcccga gtcttgcctc ggcggcgggc gctctgctca     360

tggagcgcgc gtccggggac cttgcacaga tagcgtggtc cggccagacg acgaggcttg     420

caggatcata atcagccata ccacatttgt agaggtttta cttgctttaa aaaacctccc     480

acacctcccc ctgaacctga aacataaaat gaatgcaatt gttgttgtta acttgtttat     540

tgcagcttat aatggttaca aataaagcaa tagcatcaca aatttcacaa ataaagcatt     600

tttttcactg cattctagtt gtggtttgtc caaactcatc aatgtatctt atcatgtctg     660

gatcacgatg cggccgcgct agtgtcgacc ctccatcaga gatgtgtctt gtcgcaggat 720

ttcggctcca ctttcttgtc caccttcgta ttggagggtt tgtggttgac attgcagatg 780

taggtctggg tccctaagct ggaggaaggc acagtaacca cgcttgacag agagtacaga 840

ccggatgact ggagtacggc tggaaacgta tgcacaccag aggttagagc accactattc 900

caagagacag tcactggttc tggaaagtag tccttcacca gacatcccag ggctgctgtc 960

cctccactgg tgctcttaga ggagggagcc aaggggaata cgcttgggcc tttggttgag 1020

gcggatgaca ctgtgaccag ggttccttgt ccccaaacat cgaaatacca gtgacttgtc 1080

ccgtaatagt acgggtactt tgcgcagtag tacacggcag tatcttcggc tctcagagag 1140

ttcatctgca gataggcggt tgacttgcta gtatcaaggc taaaggtgaa cctgcgttta 1200

aagtctgccg catatgtggg ctcgccagta taggtgttaa tccagcctac ccactctaac 1260

cctttgccag gagcttgtcg aacccagttc atcccatagt gtgtgaagtc atagccacta 1320

gctgcacagc tcaaccgtag gcttccgcca ggctgaacca gtccaccgcc actttcgacg 1380

agctgcacct ctcctcctcc gccgccgccg cattcacctc gattgaaaga cttggtcacg 1440

ggagagctta agccctgatg agtcacctca caggcataga ccttgtgttt ctcgtaatcg 1500

gccttactca gggtcagggt gcttgaaagg ctgtaggtgc tatccttgct gtcctgttct 1560

gtgacgcttt cctgtgaatt gccagattgg agtgcattat ccactttcca ttgcaccttc 1620

gcttctctgg gatagaagtt gttgagaagg caaaccacac ttgccgtccc ggatttcagc 1680

tgctcatctg acggtggaaa gatgaacacg ctaggagcag caacagtgcg tttgatctct 1740

acctttgttc cttgtccaaa tgtccaaggc acagtgctat actgctgaca gtagtaggtg 1800

gcaaagtcct ctggttgtaa gcttgagatg gtgagagtga agtctgtgcc actgccagat 1860

ccggaaaacc tggagggcac accactgtgc aagctggagg tgaagtagat cagcacttta 1920

ggggctttgc caggcttctg ctgataccag ttcaagtagt tggaaatgtc ctgggatgcg 1980

gaacaggtaa tcgtgacccg atctccgacg gaagcgctca gagaagaggg agactgagtc 2040

agctgtatgt catcccctgt actccctgga acccatagaa gcagtaccca caacaggagt 2100

gtgtccgtct ccatgctgca tgcgaattcg gatccccggg taccgagctc gaattcgggg 2160

ccgcggaggc tggatcggtc ccggtgtctt ctatggaggt caaaacagcg tggatggcgt 2220

ctccaggcga tctgacggtt cactaaacga gctctgctta tataggcctc ccaccgtaca 2280

cgcctactcg acccgggtac cgagctcgac tttcactttt ctctatcact gatagggagt 2340

ggtaaactcg actttcactt ttctctatca ctgataggga gtggtaaact cgactttcac 2400

ttttctctat cactgatagg gagtggtaaa ctcgactttc acttttctct atcactgata 2460

gggagtggta aactcgactt tcacttttct ctatcactga tagggagtgg taaactcgac 2520

tttcactttt ctctatcact gatagggagt ggtaaactcg actttcactt ttctctatca 2580

ctgataggga gtggtaaact cgaactagtt cgaggtcgac ggtatcgata agcttgattc 2640

gagccccagc tggttctttc cgcctcagaa gccatagagc ccaccgcatc cccagcatgc 2700

ctgctattgt cttcccaatc ctcccccttg ctgtcctgcc ccaccccacc ccccagaata 2760

gaatgacacc tactcagaca atgcgatgca atttcctcat tttattagga aaggacagtg 2820

ggagtggcac cttccagggt caaggaaggc acgggggagg ggcaaacaac agatggctgg 2880

caactagaag gcacagtcga ggctgatcag cgagctctag catttaggtg acactataag 2940

aatagggccc tctaatcgaa ttcctgcagc ccgggggatc gatccttact tagttacccg 3000

gggagcatgt caaggtcaaa atcgtcaaga gcgtcagcag gcagcatatc aaggtcaaag 3060

tcgtcaaggg catcggctgg gagcatgtct aagtcaaaat cgtcaagggc gtcggccggc 3120

ccgccgcttt cgcactttag ctgtttctcc aggccacata tgattagttc caggccgaaa 3180

aggaaggcag gttcggctcc ctgccggtcg aacagctcaa ttgcttgtct cagaagtggg 3240

ggcatagaat cggtggtagg tgtctctctt tcctcttttg ctacttgatg ctcctgttcc 3300

tccaatacgc agcccagtgt aaagtggccc acggcggaca gagcgtacag tgcgttctcc 3360

agggagaagc cttgctgaca caggaacgcg agctgatttt ccagggtttc gtactgtttc 3420

tctgttgggc gggtgccgag atgcacttta gccccgtcgc gatgtgagag gagagcacag 3480

cggaatgact tggcgttgtt ccgcagaaag tcttgccatg actcgccttc cagggggcag 3540

aagtgggtat gatgcctgtc cagcatctcg attggcaggg catcgagcag ggcccgcttg 3600

ttcttcacgt gccagtacag ggtaggctgc tcaactccca gcttttgagc gagtttcctt 3660

gtcgtcaggc cttcgatacc gactccattg agtaattcca gagcgccgtt tatgactttg 3720

ctcttgtcca gtctagacat ggtgaattca atttaaatcg taccgagcga ctcgacgcgt 3780

tcgctcgaat taatcaattc tttgccaaaa tgatgagaca gcacaataac cagcacgttg 3840

cccaggagct gtaggaaaaa gaagaaggca tgaacatggt tagcagaggc tctagagccg 3900

ccggtcacac gccagaagcc gaaccccgcc ctgccccgtc ccccccgaag gcagccgtcc 3960

ccccgcggac agccccgagg ctggagaggg agaaggggac ggcggcgcgg cgacgcacga 4020

aggccctccc cgcccatttc cttcctgccg gggccctccc ggagcccctc aaggctttca 4080

cgcagccaca gaaaagaaac aagccgtcat taaaccaagc gctaattaca gcccggagga 4140

gaagggccgt cccgcccgct cacctgtggg agtaacgcgg tcagtcagag ccggggcggg 4200

cggcgcgagg cggcgcggag cggggcacgg ggcgaaggca acgcagcgac tcccgcccgc 4260

cgcgcgcttc gctttttata gggccgccgc cgccgccgcc tcgccataaa aggaaacttt 4320

cggagcgcgc cgctctgatt ggctgccgcc gcacctctcc gcctcgcccc gccccgcccc 4380

tcgccccgcc ccgccccgcc tggcgcgcgc cccccccccc cccccgcccc catcgctgca 4440

caaaataatt aaaaaataaa taaatacaaa attgggggtg gggagggggg ggagatgggg 4500

agagtgaagc agaacgtggg gctcacctcg accatggtaa tagcgatgac taatacgtag 4560

atgtactgcc aagtaggaaa gtcccataag gtcatgtact gggcataatg ccaggcgggc 4620

catttaccgt cattgacgtc aatagggggc gtacttggca tatgatacac ttgatgtact 4680

gccaagtggg cagtttaccg taaatactcc acccattgac gtcaatggaa agtccctatt 4740

ggcgttacta tgggaacata cgtcattatt gacgtcaatg ggcgggggtc gttgggcggt 4800

cagccaggcg ggccatttac cgtaagttat gtaacgcgga actccatata tgggctatga 4860

actaatgacc ccgtaattga ttactattaa taactagttc gagatccccg ggtaccgagc 4920

tcgaattcat cgatgattag agcatggcta cgtagataag tagcatggcg ggttaatcat 4980

taactacaag gaacccctag tgatggagtt ggccactccc tctctgcgcg ctcgctcgct 5040

cactgaggcc gggcgaccaa aggtcgcccg acgcccgggc tttgcccggg cggcctcagt 5100

gagcgagcga gcgcgcagct ggcgtaatag cgaagaggcc cgcaccgatc gcccttccca 5160

acagttgcgc agcctgaatg gcgaatggcg attccgttgc aatggctggc ggtaatattg 5220

ttctggatat taccagcaag gccgatagtt tgagttcttc tactcaggca agtgatgtta 5280

ttactaatca aagaagtatt gcgacaacgg ttaatttgcg tgatggacag actcttttac 5340

tcggtggcct cactgattat aaaaacactt ctcaggattc tggcgtaccg ttcctgtcta 5400

aaatcccttt aatcggcctc ctgtttagct cccgctctga ttctaacgag gaaagcacgt 5460

tatacgtgct cgtcaaagca accatagtac gcgccctgta gcggcgcatt aagcgcggcg 5520

ggtgtggtgg ttacgcgcag cgtgaccgct acacttgcca gcgccctagc gcccgctcct 5580

ttcgctttct tcccttcctt tctcgccacg ttcgccggct ttccccgtca agctctaaat 5640

cgggggctcc ctttagggtt ccgatttagt gctttacggc acctcgaccc caaaaaactt 5700

gattagggtg atggttcacg tagtgggcca tcgccctgat agacggtttt tcgccctttg 5760

acgttggagt ccacgttctt taatagtgga ctcttgttcc aaactggaac aacactcaac 5820

cctatctcgg tctattcttt tgatttataa gggattttgc cgatttcggc ctattggtta 5880

aaaaatgagc tgatttaaca aaaatttaac gcgaatttta acaaaatatt aacgcttaca 5940

atttaaatat ttgcttatac aatcttcctg tttttggggc ttttctgatt atcaaccggg 6000

gtacatatga ttgacatgct agttttacga ttaccgttca tcgattctct tgtttgctcc 6060

agactctcag gcaatgacct gatagccttt gtagagacct ctcaaaaata gctaccctct 6120

ccggcatgaa tttatcagct agaacggttg aatatcatat tgatggtgat ttgactgtct 6180

ccggcctttc tcacccgttt gaatctttac ctacacatta ctcaggcatt gcatttaaaa 6240

tatatgaggg ttctaaaaat ttttatcctt gcgttgaaat aaaggcttct cccgcaaaag 6300

tattacaggg tcataatgtt tttggtacaa ccgatttagc tttatgctct gaggctttat 6360

tgcttaattt tgctaattct ttgccttgcc tgtatgattt attggatgtt ggaatcgcct 6420

gatgcggtat tttctcctta cgcatctgtg cggtatttca caccgcatat ggtgcactct 6480

cagtacaatc tgctctgatg ccgcatagtt aagccagccc cgacacccgc caacacccgc 6540

tgacgcgccc tgacgggctt gtctgctccc ggcatccgct tacagacaag ctgtgaccgt 6600

ctccgggagc tgcatgtgtc agaggttttc accgtcatca ccgaaacgcg cgagacgaaa 6660

gggcctcgtg atacgcctat ttttataggt taatgtcatg ataataatgg tttcttagac 6720

gtcaggtggc acttttcggg gaaatgtgcg cggaacccct atttgtttat ttttctaaat 6780

acattcaaat atgtatccgc tcatgagaca ataaccctga taaatgcttc aataatattg 6840

aaaaaggaag agtatgagta ttcaacattt ccgtgtcgcc cttattccct tttttgcggc 6900

attttgcctt cctgtttttg ctcacccaga aacgctggtg aaagtaaaag atgctgaaga 6960

tcagttgggt gcacgagtgg gttacatcga actggatctc aacagcggta agatccttga 7020

gagttttcgc cccgaagaac gttttccaat gatgagcact tttaaagttc tgctatgtgg 7080

cgcggtatta tcccgtattg acgccgggca agagcaactc ggtcgccgca tacactattc 7140

tcagaatgac ttggttgagt actcaccagt cacagaaaag catcttacgg atggcatgac 7200

agtaagagaa ttatgcagtg ctgccataac catgagtgat aacactgcgg ccaacttact 7260

tctgacaacg atcggaggac cgaaggagct aaccgctttt ttgcacaaca tgggggatca 7320

tgtaactcgc cttgatcgtt gggaaccgga gctgaatgaa gccataccaa acgacgagcg 7380

tgacaccacg atgcctgtag caatggcaac aacgttgcgc aaactattaa ctggcgaact 7440

acttactcta gcttcccggc aacaattaat agactggatg gaggcggata aagttgcagg 7500

accacttctg cgctcggccc ttccggctgg ctggtttatt gctgataaat ctggagccgg 7560

tgagcgtggg tctcgcggta tcattgcagc actggggcca gatggtaagc cctcccgtat 7620

cgtagttatc tacacgacgg ggagtcaggc aactatggat gaacgaaata gacagatcgc 7680

tgagataggt gcctcactga ttaagcattg gtaactgtca gaccaagttt actcatatat 7740

actttagatt gatttaaaac ttcattttta atttaaaagg atctaggtga agatcctttt 7800

tgataatctc atgaccaaaa tcccttaacg tgagttttcg ttccactgag cgtcagaccc 7860

cgtagaaaag atcaaaggat cttcttgaga tccttttttt ctgcgcgtaa tctgctgctt 7920

gcaaacaaaa aaaccaccgc taccagcggt ggtttgtttg ccggatcaag agctaccaac 7980

tctttttccg aaggtaactg gcttcagcag agcgcagata ccaaatactg ttcttctagt 8040

gtagccgtag ttaggccacc acttcaagaa ctctgtagca ccgcctacat acctcgctct 8100

gctaatcctg ttaccagtgg ctgctgccag tggcgataag tcgtgtctta ccgggttgga 8160

ctcaagacga tagttaccgg ataaggcgca gcggtcgggc tgaacggggg gttcgtgcac 8220

acagcccagc ttggagcgaa cgacctacac cgaactgaga tacctacagc gtgagctatg 8280

agaaagcgcc acgcttcccg aagggagaaa ggcggacagg tatccggtaa gcggcagggt 8340

cggaacagga gagcgcacga gggagcttcc agggggaaac gcctggtatc tttatagtcc 8400

tgtcgggttt cgccacctct gacttgagcg tcgatttttg tgatgctcgt caggggggcg 8460

gagcctatgg aaaaacgcca gcaacgcggc ctttttacgg ttcctggcct tttgctggcc 8520

ttttgctcac atgttctttc ctgcgttatc ccctgattct gtggataacc gtattaccgc 8580

ctttgagtga gctgataccg ctcgccgcag ccgaacgacc gagcgcagcg agtcagtgag 8640

cgaggaagcg gaagagcgcc caatacgcaa accgcctctc cccgcgcgtt ggccgattca 8700

ttaatgcagc tgcgcgctcg ctcgctcact gaggcc 8736

## Description

### Background of the invention

Most retinal neovascular disorders are caused by upregulation of vascular endothelial growth factor (VEGF) expression, which leads to an uncontrolled formation of new, immature blood vessels in the eye. VEGF is a homodimeric heparin-binding glycoprotein with pro-angiogenic properties, stimulating migration and proliferation of micro- and macrovascular endothelial cells. The VEGF family consists of at least six subgroups named VEGF-A to VEGF-E and the placental growth factor. VEGF-A is the most prominent and potent isoform and key regulator in physiological angiogenesis as well as abnormal vascularization (neovascularization [NV]).

The expression of VEGF is upregulated under hypoxic conditions and by various cytokines. Ocular diseases with NV as a typical pathological feature include the neovascular form of age-related macular degeneration (wet/exudative AMD), diabetic macular edema (DME) in patients with diabetic retinopathy, retinal vein occlusion (RVO), and retinopathy of prematurity (ROP). The neovascularization is mostly caused through the effects of VEGF, inducing a phenotypic switch of endothelial cells. VEGF-A stimulates angiogenesis and NV through the binding to VEGF receptors (VEGF-R) located on endothelial cell surfaces, thus activating intracellular signaling pathways.

An effective way to inhibit VEGF-mediated activation of VEGF-Rs is to neutralize VEGF molecules before binding to the receptor through interaction with a VEGF-binding protein (anti-VEGF). With the advent of production techniques like the phage display technology and recombinant DNA technology, the generation of anti-VEGF molecules became possible. These molecules include whole antibodies (bevacizumab, Avastin®), antigen-binding fragments [F(ab)s] (ranibizumab, Lucentis®), or soluble molecules containing parts of the receptor-binding domain of the VEGF-R (aflibercept, EYLEA®).

Ranibizumab, the humanized F(ab) of the original whole IgG antibody bevacizumab, is inactivating VEGF due to the binding to the receptor-binding sites of all VEGF-A isoforms. It was uniquely designed for the treatment of NV in the eye and is FDA and EMA approved for use in AMD, DME, and RVO.

The pivotal disadvantage of all anti-VEGF molecules is the short half-life in the human eye (e.g., t1/2 = 7.19 days for ranibizumab). This impedes repeated injections to provide sustained VEGF blockade. However, such repeated injections of anti-VEGF molecules are very expensive and may have severe side effects like intraocular inflammation or retinal detachment. As to the state of the art, therapeutic interventions involving administration of anti-VEGF molecules are not correlated so far with the actual prevalent VEGF concentration but are solely based on the diagnosis of a retinal neovascular disorder.

According to the state of the art, tetracycline-inducible (TetOn) vectors have been constructed that encode single chain variable fragments (scFv) of anti-VEGF molecules (anti-VEGF-scFv), like for example Ra02, which is based on Ranibizumab and expressed as one single molecule.

As known from the state of the art, it is possible to measure VEGF concentration ex vivo, either with samples from blood or from aqueous liquid that was received by dotting of the eye, using enzyme-linked immunosorbent assay (ELISA) formats. However, these methods have some major disadvantages: First, it is not clear whether the VEGF concentration in blood correlates with the VEGF concentration in the eye; second, dotting of the eye may cause severe afflictions like endophthalmitis that may lead to complete loss of sight; and third, all commercially available ELISA formats need high sample volumes (> 5 µl) and have a detection limit in the range of picogram per milliliter (pg/ml). Plasma VEGF concentrations in healthy volunteers and patients with neovascular disorder and different forms of cancer are often below the current limit of detection (5-10 pg/ml). However, this factor is crucial in the pathogenesis of these disorders and precise knowledge about the concentrations in the circulation is crucial. A method for minimally invasive in vivo measurement of VEGF concentration in the eye of patients with retinal neovascular disorders is so far not known.

### Technical problem

There is a need for a minimally invasive method for determination of intraocular VEGF concentration that allows for a decision if therapeutic intervention with anti-VEGF antibodies of patients with a retinal disorder, like age-related macular degeneration, diabetic macular edema, diabetic retinopathy, retinopathy of prematurity, or retinal vein occlusion, is necessary due to the measured prevalent VEGF concentration. Moreover, there is a need for a method combining both, minimally invasive intraocular in vivo measurement of VEGF concentration and control of VEGF concentration by synthesis of anti-VEGF molecules in vivo that is dependent on the measured VEGF concentration.
Furthermore, the state of the art lacks a highly sensitive assay in the range of femtogram per milliliter (fg/ml) for in vitro determination of VEGF concentration with a small sample volume.

### Solution of the problem

The aforementioned technical problem is solved by a method using a biosensor that provides an increased fluorescent BRET signal upon binding of VEGF. For measurement of VEGF concentration in vivo, the biosensor is encapsulated in an eye-implantable, permeable microcapsule, microparticle, microbead, or gel. Additionally, for control of VEGF concentration in vivo, a doxycycline-inducible vector for synthesis of anti-VEGF molecules that is transduced into eukaryotic cells is also encapsulated therein. Moreover, the invention provides a method for highly sensitive determination of VEGF concentration in the range of femtogram per milliliter in vitro with a small sample volume of 1 to 10 µl.

### Description of the invention

In one aspect, the invention provides a method for measurement and control of intraocular VEGF concentration, which comprises the following steps:
- addition of a Renilla luciferase substrate to VEGF-binding biosensor molecules each comprising an anti-VEGF single chain variable fragment (anti-VEGF-scFv) with Renilla luciferase fused to its N-terminus and a fluorescent protein or peptide fused to its C-terminus in a liquid containing VEGF,
- measurement of bioluminescence resonance energy transfer (BRET) signal depending on binding of prevalent VEGF to the biosensor molecules as an indicator for VEGF concentration,
- induction of expression of anti-VEGF molecules by addition of doxycycline to a vector encoding anti-VEGF molecules that is transduced into eukaryotic cells.

According to the present invention, in the first step of the method prevalent VEGF molecules bind to the VEGF-binding biosensor molecules, which are chimeric proteins that comprise each an anti-VEGF single chain variable fragment (anti-VEGF-scFv, VEGF binding domain) with Renilla luciferase fused to its N-terminus and a fluorescent protein or peptide fused to its C-terminus. Such biosensors are known from the state of the art.

In a preferred embodiment, the anti-VEGF-scFv is Ra02, which is derived from Ranibizumab, the Renilla luciferase is Renilla luciferase mutant 8 (RLuc8), and the fluorescent protein is GFP2, YFP, eYFP, TurboYFP, or a peptide or derivative or mutant thereof; all of these molecules are known to the skilled worker.

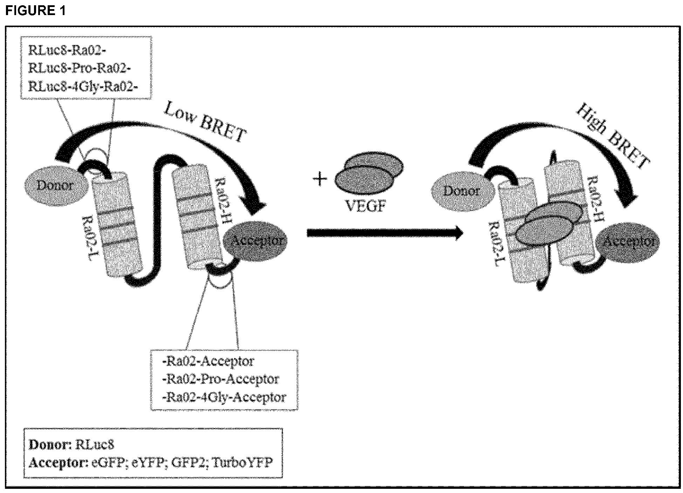

If VEGF is present, it binds to the anti-VEGF-scFv part of the chimeric protein. Subsequently, binding of VEGF triggers a conformational change (ligand-induced conformational rearrangement) of the chimeric protein. In the presence of Renilla luciferase substrate Coelenterazine, e.g. EnduRen™ or ViviRen™, this conformational change is generating an increase of a BRET signal, which is mediated through radiationless energy transfer, based on dipole-dipole interaction, from the N-terminally located Renilla luciferase as the signal donor to the C-terminally located fluorescent protein as the signal acceptor. The BRET signal is a quotient of the intensity of the emitted radiation of the fluorescent protein and the intensity of the emitted radiation of the Renilla luciferase that is due to substrate conversion (Figure 1). The intensity of the BRET signal directly correlates with the VEGF concentration: a higher VEGF concentration leads to a higher BRET signal. The BRET technology is described in the state of the art.

For performance of the method in vivo, i.e. in the eye of a patient with a retinal neovascular disorder, the VEGF-binding biosensor as described herein according to the present invention is encapsulated in an insert like a microcapsule, microparticle, microbead, or gel, that is permeable for VEGF, Renilla luciferase substrate, doxycycline, and anti-VEGF molecules, but is not permeable for VEGF-anti-VEGF antigen-antibody complexes or VEGF that is bound to the VEGF-binding biosensor.

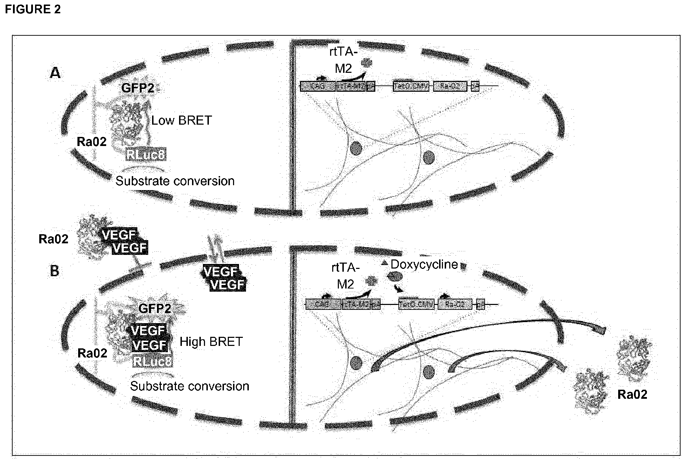

In a preferred embodiment, the insert is made from alginate. This insert can be implanted into the eye of the patient, which enables a permanent, minimally invasive measurement of VEGF concentration directly in the eye via BRET signal using a device that is able to detect the BRET signal through the vitreous body and the front part of the eye (Figure 2). Such devices are commercially available which use an appropriate software, like ImageJ, for instance, to analyze the image data. For in vivo measurement, the Renilla luciferase substrate Coelenterazine is administered intravenously or orally to the patient. Based on the measured VEGF concentration, a decision can be made if a therapeutic intervention by administration of anti-VEGF molecules is necessary or not (Example 1).

In order to enable a minimally invasive therapy avoiding dotting of the eye, a vector encoding anti-VEGF molecules that is transduced into a eukaryotic cell line is additionally encapsulated in the insert (Figure 2, SEQ ID No. 2). In a preferred embodiment, the vector encoding anti-VEGF molecules is TetOn-Ra02 (SEQ ID No. 2) as known from the state of the art. Expression of anti-VEGF molecules like Ra02 from this vector is induced by addition of a tetracycline, preferably doxycycline. In a preferred embodiment, doxycycline is administered orally to the patient at doses as described in the state of the art (Example 2).

For performance of the method in vitro, a vector encoding a VEGF-binding biosensor according to the invention is transfected into eukaryotic cells. In a preferred embodiment, the eukaryotic cells are HEK-293 cells and the VEGF-binding biosensor is RLuc8-Ra02-GFP2 (SEQ ID No. 1). In the cells, the biosensor is expressed within 48 hours and can be subsequently isolated from the cells, as described in example 3. Afterwards, an aliquot of the biosensor sample is incubated with samples from which the VEGF concentration has to be determined. These samples are from blood or from aqueous liquid of the eye of a patient whose VEGF concentration in the eye should be determined. In parallel, the biosensor is incubated with appropriate positive controls (VEGF serial dilution) or negative controls (PBS, RLuc8-Ra02). After addition of Renilla luciferase substrate Coelenterazine, the BRET ratio is measured with a plate reader in dual luminescence mode using the filter sets magenta and green, the ratio is normalized with the negative samples, and the VEGF concentration of the samples is determined by linear regression (Example 3, Figure 1).

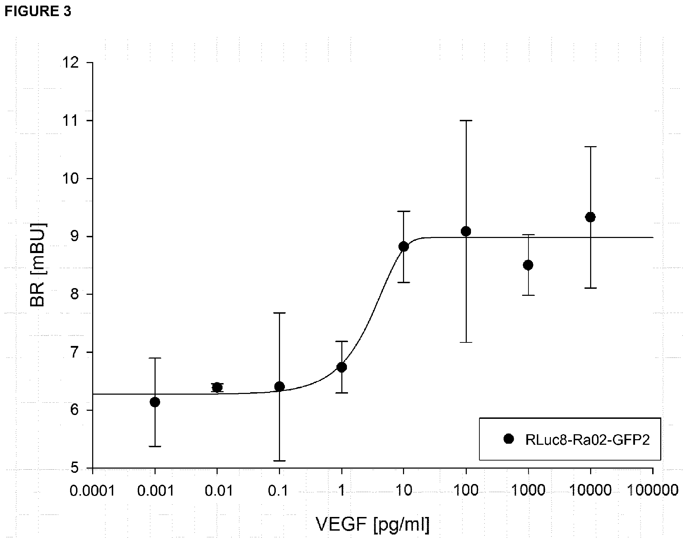

Surprisingly it is found in the present invention, that much lower VEGF concentrations can be measured by use of the VEGF-binding biosensor as described herein as with assays known from the state of the art. The new method is suitable for measurement of VEGF concentrations from 100 fg/ml or up to 10 ng/ml (Figure 3), whereas commercially available assays have a detection limit of at least 1.7 pg/ml.

### Examples

The examples below illustrate the present invention.

### Example 1: Determination of intraocular VEGF concentration in vivo

According to the present invention, the intraocular VEGF concentration in vivo is determined via the following steps. First, the substrate for Renilla luciferase, Coelenterazine, is administered to the patient whose VEGF concentration should be measured in the eye and to whom the VEGF-binding biosensor, for example RLuc8-Ra02-GFP2 (SEQ ID No.1), or RLuc8-Ra02-YFP, or RLuc8-Ra02-eYFP, or RLuc8-Ra02-TurboYFP, encapsulated in an insert, e.g. a microbead made from alginate, according to the present invention (Figure 1, Figure 2) has been implanted previously into the eye. Coelenterazine is commercially available, e.g. as EnduRen™ or ViviRen™ Live Cell Substrate (Promega), and is administered intravenously or orally, respectively, at doses that are known to the skilled worker. Subsequently, at an appropriate time after administration, the BRET ratio is measured using filters absorbing light at wavelengths of below 450 nm (signal from Renilla luciferase) and 500-550 nm (BRET signal from fluorescent protein or peptide), respectively, in a device for detection of luminescence using an appropriate image analysis software, like e.g. ImageJ. Such devices and softwares are known to the skilled worker. The measured delta BRET ratio correlates with the prevalent VEGF concentration in the eye of the patient and is determined from the BRET ratio by linear regression.

### Example 2: Induction of anti-VEGF synthesis in vivo

According to the invention described herein, the insert harbouring the VEGF-binding biosensor may additionally encapsulate a tetracycline-inducible (TetOn) vector encoding anti-VEGF molecules that is transduced into HEK-293 cells (Figure 2). This vector is TetOn-Ra02 that has been previously described (Wimmer et al., Functional Characterization of AAV-Expressed Recombinant Anti-VEGF Single-Chain Variable Fragments In Vitro. J Ocul Pharmacol Ther. 2015 Jun;31 (5):269-76) (SEQ ID No. 2). Expression of anti-VEGF molecules from TetOn-Ra02 is induced by oral administration of doxycycline to the patient with doses according to the state of the art (0.5 to 10 mg/kg body weight). The newly synthesized anti-VEGF molecules bind to VEGF that is present in the eye and therefore reduces the concentration of free VEGF. The reduction of VEGF concentration can be determined at an appropriate time after administration of doxycycline by the method described in example 1 according to the present invention.

### Example 3: Determination of intraocular VEGF concentration in vitro

According to the invention presented herein, for determination of intraocular VEGF concentration in vitro, in a first step for example 6 µg of a vector encoding VEGF-binding biosensor molecules, for example RLuc8-Ra02-GFP2 (SEQ ID No. 1), are transfected in a 6-well with Lipofectamine 3000 (Invitrogen) into eukaryotic HEK-293 cells. This method is known to the skilled worker. RLuc8-Ra02-GFP2 comprises an anti-VEGF single chain variable fragment (anti-VEGF-scFv; Ra02) with Renilla luciferase (RLuc8) fused to its N-terminus and a fluorescent protein (GFP2) fused to its C-terminus. Afterwards, expression of RLuc8-Ra02-GFP2 biosensor molecules is allowed for 48 hours in an incubation chamber at 37°C and 5% CO₂. Expression is then verified by luciferase activity assay and fluorescence microscopy; both techniques are known from the state of the art. Subsequently, the newly synthesized biosensor molecules RLuc8-Ra02-GFP2 are isolated from the HEK-293 cells by use of 150 µl per well Renilla Luciferase Assay Lysis Buffer (Promega) followed by two steps of freezing in liquid nitrogen and thawing. Afterwards the samples are centrifuged for 5 min at 14,000 x g and 4°C in order to get rid of any left cell particles. The soluble fraction then harbors the biosensor molecules. In parallel, serial dilutions ranging from 10 ng/ml down to 1 fg/ml of VEGF are made in phosphate-buffered saline (PBS) as positive control. Furthermore, serial dilutions in PBS are made with 1 or up to 10 µl of samples from which the VEGF concentration shall be determined. For determination of the VEGF concentration, 10 µl of the biosensor fraction is incubated at 4°C overnight with 10 µl each of either the serial VEGF dilutions as positive controls, or the diluted samples from which the VEGF concentration should be determined, or PBS as a negative control, or a RLuc8-Ra02 construct lacking GFP2 at the C-terminus as negative control. Afterwards, 100 µl of the Renilla luciferase substrate Coelenterazine 400a (Nanolight, Inc.; 63 µM) is added to each of the samples to start the BRET assay. The BRET ratio is measured with a plate reader (Tecan Infinite 1000Pro) in dual luminescence mode using the filter sets magenta and green. The BRET ratio is normalized using the negative sample (delta BRET ratio). The correlating VEGF concentration, which is determined by linear regression, is displayed on the x-axis, and the delta BRET ratio is shown on the y-axis.
As can be seen in Figure 3, dependent on the VEGF concentration, the BRET ratio is 0.785 ± 0.039. The VEGF binding capacity per 1x10⁶ RLU (relative light units) is 20.29 pg ± 6.60 pg. The linear range of the VEGF dependent BRET ratio change is 100 fg/ml up to 10 ng/ml for the RLuc8-Ra02-GFP2 biosensor.

### Description of the drawings

**Figure 1****.** Antigen-induced conformational change of the VEGF-binding biosensor according to the present invention and BRET assay. If VEGF molecules are present, VEGF binds to the Ra02 parts (Ra02-L and Ra02-H) of the biosensor molecule and therefore induces a conformational change of the biosensor molecule. Thus, in the presence of Renilla luciferase substrate Coelenterazine, the Renilla luciferase RLuc8 at the N-terminus of the biosensor molecule emits radiation as a donor (BRET signal 1) that is then accepted by a fluorescent protein or peptide, e.g. GFP2, eGFP, eYFP, or TurboYFP, at the C-terminus of the biosensor molecule. The acceptor molecule then emits itself radiation at another wavelength than the donor (BRET signal 2). Both BRET signals are measured using a device using appropriate filters (e.g. magenta for BRET signal 1 and green for BRET signal 2) and a software that determines the BRET ratio. Then the BRET ratio is used for determination of the VEGF concentration by linear regression methods. The different parts of biosensor molecule (RLuc8, Ra02, and the fluorescent protein or peptide) are either fused directly to each other or are separated by proline (Pro) or 4x glycine (4Gly) linkers.
**Figure 2****.** Insert for implantation into the eye of a patient whose VEGF concentration in the eye should be measured in vivo. The insert is a microcapsule, microparticle, microbead, or gel, for example made from alginate, that is permeable for VEGF, Renilla luciferase substrate Coelenterazine, doxycycline, and anti-VEGF molecules, but is not permeable for VEGF-anti-VEGF antigen-antibody complexes and VEGF bound to the VEGF-binding biosensor. The insert encapsulates VEGF-binding biosensor molecules, like e.g. RLucB-Ra02-GFP2 (SEQ ID No. 1), and additionally a doxycycline-inducible vector, TetOn-Ra02, that encodes anti-VEGF molecules and which is transduced into eukaryotic cells. **A** In the absence of free VEGF molecules, RLuc8 may convert its substrate Colenterazine and emit radiation, but this radiation can be transferred via BRET to the acceptor GFP only at a very low level. **B** In the presence of free VEGF molecules, the biosensor molecules undergoes a conformational change upon binding of VEGF to Ra02 of the biosensor. As a consequence, in the presence of Coelenterazine, the radiation of RLuc8 is transferred to the acceptor GFP, which then itself emits radiation. Both signals can be measured with an appropriate device and the BRET ratio as well as the VEGF concentration can be determined as described herein. If the VEGF concentration is too high, synthesis of anti-VEGF molecules can be triggered by administration of doxycycline to the patient. Doxycycline then induces expression of anti-VEGF molecules from the vector TetOn-Ra02 in the eukaryotic cells that are also encapsulated in the insert. Newly synthesized anti-VEGF molecules are small enough to leave the insert and to bind to free VEGF that is present in the eye of the patient.
**Figure 3****.** Change of BRET ratio in dependence of the VEGF concentration. The VEGF concentration is displayed on the x-axis (c(VEGF)) at ng/ml. On the y-axis the change of the BRET ration dependent on the VEGF concentration is shown in delta milli BRET Units (mBU). Exponential growth is measured in the range of 0.0001 ng/ml up to 0.01 ng/ml which corresponds to 2 log units of the VEGF concentration.

The following sequences referred to herein are shown in the accompanying sequence listing:

### Sequence Listing

**SEQ ID No. 1:** RLuc8-Ra02-GFP2 biosensor molecule
**SEQ ID No. 2:** Vector with TetOn-Ra02 expression cassette

## Claims

1. Method for measurement and control of intraocular vascular endothelial growth factor (VEGF) concentration, which comprises the following steps:
- addition of a Renilla luciferase substrate to VEGF-binding biosensor molecules each comprising an anti-VEGF single chain variable fragment (anti-VEGF-scFv) with Renilla luciferase fused to its N-terminus and a fluorescent protein or peptide fused to its C-terminus in a liquid containing VEGF,
- measurement of bioluminescence resonance energy transfer (BRET) signal depending on binding of prevalent VEGF to the biosensor molecules as an indicator for VEGF concentration,
- induction of expression of anti-VEGF molecules by addition of doxycycline to a vector encoding anti-VEGF molecules that is transduced into eukaryotic cells.

2. Method according to claim 1, wherein the fluorescent protein is GFP2, YFP, eYFP, TurboYFP, or peptides or derivatives or mutants thereof.

3. Method according to claims 1 to 2, wherein measurement and control of intraocular VEGF concentration are performed in vivo.

4. Method according to claims 1 to 3, wherein the VEGF-binding biosensor and the vector encoding anti-VEGF molecules that is transduced into eukaryotic cells are encapsulated in an eye-implantable, permeable microcapsule, microparticle, microbead, or gel.

5. Method according to claim 4, wherein the eye-implantable, permeable microcapsule, microparticle, microbead, or gel is permeable for VEGF, Renilla luciferase substrate, doxycycline, and anti-VEGF molecules, but is not permeable for VEGF-anti-VEGF antigen-antibody complexes and VEGF bound to VEGF-binding biosensor according to claims 1 to 2.

6. Method according to claim 5, wherein the eye-implantable, permeable microcapsule, microparticle, microbead, or gel is made from alginate.

7. Method according to claims 1 to 2, wherein measurement of intraocular VEGF concentration is performed in vitro and which comprises the following steps:
- addition of a Renilla luciferase substrate to VEGF-binding biosensor molecules each comprising an anti-VEGF single chain variable fragment (anti-VEGF-scFv) with Renilla luciferase fused to its N-terminus and a fluorescent protein or peptide fused to its C-terminus in a liquid containing VEGF,
- measurement of bioluminescence resonance energy transfer (BRET) signal depending on binding of VEGF to the biosensor molecules as an indicator for VEGF concentration.

8. Method according to claim 7, wherein the measurement is performed with a sample volume of 1 to 10 µl.

9. Method according to claims 7 to 8, wherein the lower detection limit of VEGF concentration is 100 fg/ml and the upper detection limit is 10 ng/ml.

10. Use of the method according to claims 1 to 6 for diagnosis and/or therapy of VEGF-concentration-related retinal neovascular disorders like e.g. age-related macular degeneration, diabetic macular edema, diabetic retinopathy, retinopathy of prematurity, or retinal vein occlusion.

11. Use of the method according to claims 7 to 9 for diagnosis of VEGF-concentration-related retinal neovascular disorders like age-related macular degeneration, diabetic macular edema, diabetic retinopathy, retinopathy of prematurity, or retinal vein occlusion.
